# EUROPEAN PATENT APPLICATION

(11) **EP 4 166 921 A1**
(43) Date of publication of application: **19.04.2023**
(21) Application number: 21826572.6
(22) Date of filing: 21.05.2021
(51) Int. Cl.: G01N 1/02, G01N 1/22

(54) **COLLECTION DEVICE**

(30) Priority: 16.06.2020 JP 2020103956
(71) Applicant: Panasonic Intellectual Property Management Co., Ltd., Osaka-shi, Osaka 540-6207 (JP)
(72) Inventor: NARIHATA, Koki, Osaka-shi, Osaka 540-6207 (JP); SASAKI, Yoshiki, Osaka-shi, Osaka 540-6207 (JP)
(74) Representative: Eisenführ Speiser
(86) International application number: PCT/JP2021/019293
(87) International publication number: WO 2021/256166

(57) **Abstract**

A collection device (10) including a container (12) into which exhaled air containing target objects (1) is introduced, a cooler (100) that reduces a temperature of the exhaled air introduced into the container (12) to generate droplets (3) containing the target object (1), and a rotating body (14) that is provided in the container (12) and that rotates. The rotating body (14) includes first blades (44a to 44h) that project in directions crossing a rotation axis X of rotating body (14). The first blades (44a to 44h) each have a filter (46) that captures the droplets (3).

## Description

### Technical Field

The present disclosure relates to a collection device that receives exhaled air containing target objects, such as pathogens, and collects the target objects contained in the exhaled air in liquid.

### Background Art

In an example of a known sampling method, fine particles contained in gas are collected by using a device that utilizes inertia or centrifugal force of the fine particles (see, for example, PTL 1 and PTL 2).

Air exhaled by a patient infected with an infectious virus, such as an influenza virus, conceivably contains infectious viruses. The infectious viruses in the air exhaled by the patient may spread through atmospheric air in the form of particles (droplets or droplet nuclei) and be transmitted by droplet infection or airborne infection.

The route of infection, for example, can be determined by collecting the viruses from the exhaled air or atmospheric air. Quick determination of presence or absence of a virus in the exhaled air or atmospheric air may enable prevention of secondary infections.

A device for collecting a component of air exhaled by a human have been developed (see, for example, PTL 1). The device according to PTL 1 includes a helical collection pipe, a collection bottle connected to a dripping port of the collection pipe, and a cooling container for cooling the collection pipe and the collection bottle.

A virus collector capable of collecting viruses contained in air exhaled by a patient and providing the collected viruses as a sample for diagnosis or research has also been devised (see, for example, PTL 2). The virus collector according to PTL 2 includes a main body, an exhaled-air introduction portion through which the air exhaled by the patient is introduced, a trap portion that captures viruses contained in the air exhaled by the patient, and a suction portion that sucks in a gas component including the air exhaled by the patient.

### Citation List

### Patent Literature

PTL 1: Japanese Unexamined Patent Application Publication No. 7-103974
PTL 2: Japanese Unexamined Patent Application Publication No. 2008-119552

### Summary of Invention

In the device according to PTL 1, when a person exhales air into the collection pipe from an end of the collection pipe, the exhaled air flows through the collection pipe while being cooled, and a component of the exhaled air adheres to an inner wall of the collection pipe. Then, the collection pipe is removed from the cooling device and heated, so that liquid containing the component that has adhered to the inner wall accumulates in the collection bottle. However, according to PTL 1, the component of the exhaled air cannot be easily collected because the collection pipe needs to be cooled and then heated.

In the virus collector according to PTL 2, the air exhaled by the patient is introduced through the exhaled-air introduction portion having a constricted section, and a pressure drop that occurs when the exhaled air is discharged through an opening of the constricted section is used to condense vapor in the exhaled air into liquid. Viruses contained in the liquid are introduced into an isolation medium disposed in the trap portion. However, it is difficult to reliably capture the viruses in the trap portion. PTL 2 also describes a cylindrical member composed of a permeable fabric disposed between the opening of the constricted section and the trap portion to collect the liquid containing the viruses. However, a troublesome process of removing and centrifuging the permeable fabric, for example, is performed to separate the liquid containing the viruses.

The present disclosure has been made in light of the above-described problems, and provides a collection device capable of easily collecting target objects contained in exhaled air in liquid.

A collection device according to an aspect of the present disclosure includes a container into which exhaled air containing target objects is introduced; a cooler that reduces a temperature of the exhaled air introduced into the container to generate droplets containing the target objects; and a rotating body that is provided in the container and that rotates. The rotating body includes a first blade that projects in a direction crossing a rotation axis of the rotating body. The first blade includes at least one of a filter that captures the droplet and a plate surface that does not allow the droplet to pass therethrough.

The collection device according to the aspect of the present disclosure is capable of easily collecting the target objects contained in the exhaled air in liquid. Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

### Brief Description of Drawings

[Fig. 1] Fig. 1 is a perspective view of a collection device according to a first embodiment.
[Fig. 2] Fig. 2 is a sectional view taken along line II-II in Fig. 1.
[Fig. 3] Fig. 3 is a sectional view taken along line III-III in Fig. 1.
[Fig. 4] Fig. 4 is a sectional view of a collection device according to a second embodiment as viewed from the left.
[Fig. 5] Fig. 5 is a sectional view of the collection device according to the second embodiment as viewed from the front.
[Fig. 6] Fig. 6 is a sectional view of a collection device according to a third embodiment as viewed from the left.
[Fig. 7] Fig. 7 is a side view of a collection device according to a fourth embodiment as viewed from the left.
[Fig. 8] Fig. 8 is a side view of a collection device according to a fifth embodiment.

### Description of Embodiments

Embodiments of the present invention will be described with reference to the drawings.

The collection device according to the present disclosure is not intended to be limited to structures illustrated in the embodiments and/or drawings described below, and includes structures equivalent thereto.

The embodiments described below are comprehensive or specific examples. Numerical values, shapes, materials, constituent elements, positions of and connections between the constituent elements, steps, sequence of the steps, etc. are examples and are not intended to limit the scope of the claims.

The drawings are not necessarily exact illustrations. In each drawing, substantially identical components are denoted by the same reference signs, and redundant descriptions thereof may be omitted or simplified.

In the following description, terms representing the relationships between elements, such as "parallel" and "perpendicular", terms representing the shapes of elements, such as "cylindrical", and numerical ranges are not to be interpreted strictly, but rather as including substantially equivalent ranges, for example, ranges with a tolerance of several percent.

### [First Embodiment]

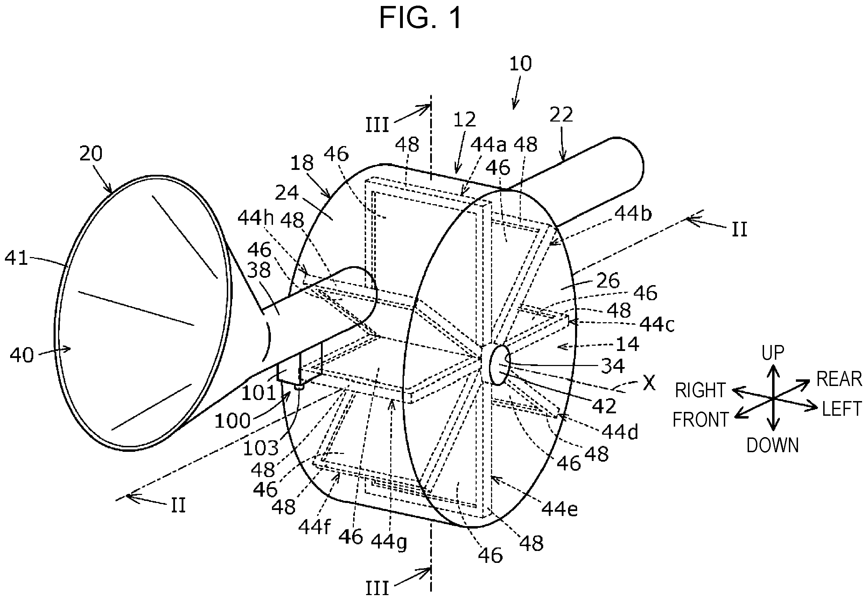

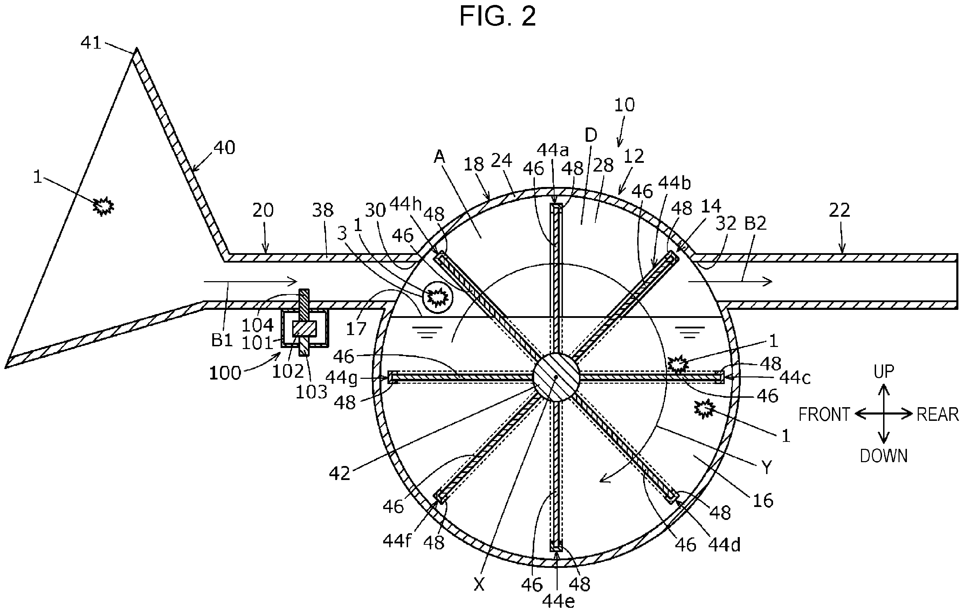

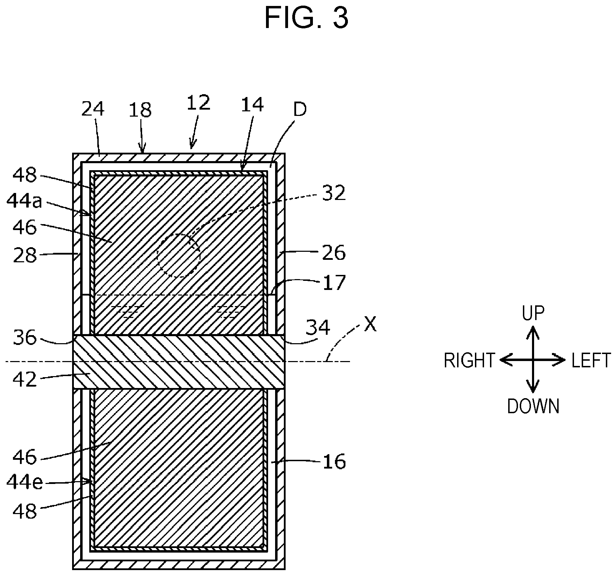

Fig. 1 is a perspective view of a collection device 10 according to a first embodiment. Fig. 2 is a sectional view taken along line II-II in Fig. 1. Fig. 3 is a sectional view taken along line III-III in Fig. 1. The collection device 10 according to the first embodiment will be described with reference to Figs. 1 to 3. In Fig. 1, collection liquid 16 (described below) is not illustrated to simply the drawing. In Fig. 3, first blades 44b and 44d (described below) are not illustrated to simply the drawing. In this embodiment, a direction from a peripheral wall 24 (described below) of a main body 18 (described below) toward an introduction pipe 20 (described below) is referred to as forward, and a direction from the peripheral wall 24 toward a discharge pipe 22 (described below) as rearward (see arrows in Figs. 1 and 2). In addition, in this embodiment, a direction from the peripheral wall 24 toward a left wall 26 (described below) is referred to as leftward, and a direction from the peripheral wall 24 toward a right wall 28 (described below) as rightward (see arrows in Figs. 1 and 3). In addition, in this embodiment, a direction from a shaft 42 (described below) toward a flow path A is referred to as upward, and a direction from the flow path A toward the shaft 42 as downward (see arrows in Figs. 1 and 3). The front-rear direction, left-right direction, and up-down direction are perpendicular to each another.

Referring to Figs. 1 to 3, the collection device 10 collects target objects 1 contained in exhaled air in liquid. Examples of the target objects 1 include pathogens, viruses, pollen, and particulate matters. The collection device 10 includes a container 12, a rotating body 14, the collection liquid 16, and a cooler 100. Each component will be described.

Gas, such as exhaled air, is introduced into the container 12. The container 12 holds the collection liquid 16 therein, and has the flow path A through which the gas flows above the collection liquid 16 held in the container 12. The container 12 supports the rotating body 14 such that the rotating body 14 is rotatable about a rotation axis X (see arrow Y in Fig. 2) in the container 12. The rotation axis X extends in the left-right direction. In other words, the rotation axis X extends perpendicularly to the up-down direction. The rotation axis X does not necessarily extend perpendicularly to the up-down direction as long as the rotation axis X extends in a direction crossing the up-down direction. The container 12 includes the main body 18, the introduction pipe 20, and the discharge pipe 22.

The main body 18 is a portion that holds the collection liquid 16 and supports the rotating body 14 such that the rotating body 14 is rotatable about the rotation axis X. The main body 18 is cylindrical, and includes the peripheral wall 24, the left wall 26, and the right wall 28. The main body 18 has a space D therein that accommodates the rotating body 14.

The peripheral wall 24 is formed around the rotation axis X. The peripheral wall 24 extends in the left-right direction, and is cylindrical. The peripheral wall 24 is provided to surround the rotating body 14. The peripheral wall 24 has a hole 30 and a hole 32 that extend through the peripheral wall 24 in the front-rear direction at positions above the rotation axis X. The hole 30 is disposed in front of the rotation axis X, and the hole 32 is disposed behind the rotation axis X. The hole 30 and the hole 32 face each other in the front-rear direction. Each of the left wall 26 and the right wall 28 extends perpendicularly to the rotation axis X, and is substantially disc-shaped. The left wall 26 and the right wall 28 face each other in the left-right direction. The left wall 26 is connected to the left end of the peripheral wall 24 to cover the left side of the rotating body 14. The left wall 26 has a circular hole 34 that extends through a central portion of the left wall 26 in the left-right direction. The right wall 28 is connected to the right end of the peripheral wall 24 to cover the right side of the rotating body 14. The right wall 28 has a circular hole 36 that extends through a central portion of the right wall 28 in the direction of the rotation axis X.

The space D is defined by the peripheral wall 24, the left wall 26, and the right wall 28. The collection liquid 16 is contained in a portion of the space D, and a portion of the rotating body 14 is immersed in the collection liquid 16. A portion of the space D that is above the collection liquid 16 constitutes a portion of the flow path A.

Gas, such as air exhaled by a user of the collection device 10 or air surrounding the collection device 10, is introduced through the introduction pipe 20, which is connected to the main body 18. The user is, for example, a patient having pathogens. The introduction pipe 20 is tubular and communicates with the space D in the main body 18. The introduction pipe 20 includes a cylindrical portion 38 and a conical tubular portion 40. The cylindrical portion 38 extends in the front-rear direction, and is cylindrical. The cylindrical portion 38 is connected to the peripheral wall 24 so as to communicate with the hole 30. The conical tubular portion 40 gradually widens toward the front, and has a conical tubular shape. The conical tubular portion 40 is connected to an end of the cylindrical portion 38 opposite to the end at which the peripheral wall 24 is connected.

The gas introduced through the introduction pipe 20 is discharged through the discharge pipe 22. The discharge pipe 22 is connected to the main body 18 at a side opposite to the side at which the introduction pipe 20 is connected. The discharge pipe 22 extends in the front-rear direction, and is cylindrical. The discharge pipe 22 is connected to the peripheral wall 24 so as to communicate with the hole 32.

The container 12 has the flow path A defined by the introduction pipe 20, the main body 18, and the discharge pipe 22. The gas introduced into the container 12 flows through the flow path A. The gas introduced into the introduction pipe 20 passes through the conical tubular portion 40 and the cylindrical portion 38 (see arrow B1 in Fig. 2), and flows into the main body 18 through the hole 30. The gas that has flowed into the main body 18 travels above the collection liquid 16 and flows into the discharge pipe 22 through the hole 32 (see arrow B2 in Fig. 2). Then, the gas is discharged through the discharge pipe 22. Thus, the container 12 has the flow path A through which the gas flows in the front-rear direction above the collection liquid 16. In other words, when viewed in the up-down direction, the container 12 has the flow path A through which the gas flows perpendicularly to the rotation axis X above the collection liquid 16. The container 12 does not necessarily have the flow path A through which the gas flows perpendicularly to the rotation axis X when viewed in the up-down direction. For example, the container 12 may instead have the flow path A through which the gas flows in a direction crossing the rotation axis X when viewed in the up-down direction.

The rotating body 14 is provided in the container 12 and rotates. The rotating body 14 is supported by the container 12 such that the rotating body 14 is rotatable about the rotation axis X. The rotating body 14 includes the shaft 42 and first blades 44a to 44h.

The shaft 42 extends in the left-right direction, and is solid cylindrical. The shaft 42 has an axial center that coincides with the rotation axis X. The shaft 42 is provided at the center of the cylindrical main body 18. More specifically, the shaft 42 has a left end portion that is rotatably fitted to the hole 34 in the left wall 26 and a right end portion that is rotatably fitted to the hole 36 in the right wall 28. Thus, the shaft 42 is supported by the main body 18 such that the shaft 42 is rotatable about the rotation axis X.

The first blades 44a to 44h project in directions crossing the rotation axis X of the rotating body 14, and are attached to the outer peripheral surface of the shaft 42 at equal intervals around the rotation axis X. In other words, the first blades 44a to 44h project outward from the outer peripheral surface of the shaft 42 in radial directions with respect to the shaft 42, and are arranged at equal intervals in the circumferential direction of the shaft 42. The first blades 44a to 44h are rotatable about the rotation axis X together with the shaft 42. The first blade 44a includes a filter 46 and a frame 48.

The filter 46 extends in the left-right direction and in a direction crossing the rotation axis X, and is rectangular-plate-shaped. It is not necessary that the filter 46 be rectangular-plate-shaped. The filter 46 projects in the direction crossing the rotation axis X and is connected to the outer peripheral surface of the shaft 42. When the rotating body 14 rotates about the rotation axis X, the filter 46 also rotates about the rotation axis X. For example, the filter 46 captures the target objects 1 contained in the gas by coming into contact with the gas. For example, the filter 46 allows the gas to pass therethrough, and captures the target objects 1 contained in the gas that passes therethrough. For example, the filter 46 captures droplets 3 generated by the cooler 100. More specifically, the filter 46 captures the droplets 3 by coming into contact with the droplets 3 that are generated by the cooler 100 and that float in the container 12. The filter 46 is, for example, net-shaped. Various known filters capable of capturing the target objects 1 in the gas may be used as the filter 46. Various known filters capable of capturing the droplets 3 may be used as the filter 46. For example, the filter 46 may be a filter composed of nonwoven fabric. The filter 46 may be, for example, a filter having a porosity such that when the gas flows through the flow path A, the gas passes through the filter and also causes the rotating body 14 to rotate about the rotation axis X.

When the filter 46 is positioned directly above the rotation axis X (position of the first blade 44a in Fig. 2), the filter 46 projects upward from a liquid surface 17 of the collection liquid 16, and is disposed in the flow path A. More specifically, when the filter 46 is positioned directly above the rotation axis X, a portion of the filter 46 is in the flow path A, and another portion of the filter 46 is immersed in the collection liquid 16. When the filter 46 is positioned directly above the rotation axis X, the filter 46 overlaps the hole 30 and the hole 32 in the front-rear direction. When the filter 46 is positioned directly below the rotation axis X (position of the first blade 44e in Fig. 2) or horizontally adjacent to the rotation axis X (positions of the first blade 44c and the first blade 44g in Fig. 2), the filter 46 is positioned below the liquid surface 17 of the collection liquid 16 and is immersed in the collection liquid 16. More specifically, when the filter 46 is positioned directly below the rotation axis X and when the filter 46 is positioned horizontally adjacent to the rotation axis X, the entirety of the filter 46 is immersed in the collection liquid 16. The filter 46 is movable from a position in the flow path A to a position immersed in the collection liquid 16 when the rotating body 14 rotates about the rotation axis X. In other words, when the rotating body 14 rotates about the rotation axis X, the filter 46 moves from a position that is not immersed in the collection liquid 16 to a position immersed in the collection liquid 16. More specifically, the filter 46 is movable from a position in the flow path A to a position immersed in the collection liquid 16 by rotating about the rotation axis X as the rotating body 14 rotates about the rotation axis X. Still more specifically, when the rotating body 14 rotates about the rotation axis X, a part of the filter 46 that has been disposed in the flow path A is immersed into the collection liquid 16. In other words, when the rotating body 14 rotates about the rotation axis X, a part of the filter 46 that has not been immersed in the collection liquid 16 is immersed into the collection liquid 16.

The frame 48 opens toward the shaft 42, and is substantially U-shaped. The frame 48 extends along the edges of the filter 46. The frame 48 is attached to the outer peripheral surface of the shaft 42. The frame 48 clamps the edges of the filter 46. The frame 48 is spaced from the inner surface of the main body 18. More specifically, the frame 48 is spaced from the inner surface of the peripheral wall 24 in a direction perpendicular to the rotation axis X. The frame 48 is spaced from the inner surface of the left wall 26 and from the inner surface of the right wall 28 in the left-right direction.

The structure of each of the first blades 44b to 44h is similar to that of the first blade 44a. Therefore, the first blades 44b to 44h may be understood by referring to the above description of the first blade 44a, and detailed description thereof is thus omitted.

The collection liquid 16 is used to collect the target objects 1, and is contained in the main body 18. More specifically, the collection liquid 16 is contained in the main body 18 such that the liquid surface 17 of the collection liquid 16 is formed above the rotation axis X and the shaft 42. The collection liquid 16 is contained in the main body 18 such that the liquid surface 17 of the collection liquid 16 is positioned below the hole 30 and the hole 32. Thus, the height of the collection liquid 16 may be such that the collection liquid 16 does not overflow through the introduction pipe 20 or the discharge pipe 22.

The cooler 100 cools the exhaled air introduced into the container 12 to reduce the temperature of the exhaled air, so that the exhaled air is formed into droplets. Thus, the cooler 100 generates the droplets 3 containing the target objects 1. More specifically, the cooler 100 cools the exhaled air that flows through the introduction pipe 20 to generate the droplets 3 containing the target objects 1. The cooler 100 includes a housing 101, a cooling source 102, a power supply switch 103, and a heat transfer rod 104.

The housing 101 is attached to the cylindrical portion 38. The cooling source 102 is contained in the housing 101.

The cooling source 102 is a member for cooling the exhaled air introduced into the container 12. The cooling source 102 may be a cooled matter, such as ice or a refrigerant pack, an instantaneous coolant that absorbs heat when impacted, or a Peltier device operated by a power supply. The cooling source 102 uses the heat transfer rod 104 to reduce the temperature of the exhaled air introduced into the container 12. More specifically, the cooling source 102 cools the heat transfer rod 104 to reduce the temperature of the heat transfer rod 104, and thereby reduces the temperature of the exhaled air that is introduced into the container 12 and that passes by the heat transfer rod 104. For example, the cooling source 102 reduces the temperature of the heat transfer rod 104 so that the temperature of the exhaled air introduced into the container 12 is reduced to a temperature that is less than or equal to a dew point. For example, the cooling source 102 reduces the temperature of the heat transfer rod 104 so that the temperature of the exhaled air introduced into the container 12 is reduced by 1°C. When the cooling source 102 is operated by a power supply, the power supply switch 103 is used. More specifically, the power supply switch 103 is turned on to activate the cooling source 102 operated by the power supply, so that the exhaled air is cooled. The power supply switch 103 is not necessary when a cooling source that is not operated by a power supply is used.

The heat transfer rod 104 is made of a thermally conductive material, and is rod-shaped. The heat transfer rod 104 is connected to the cooling source 102 at one end thereof, and is cooled by the cooling source 102 as described above. The other end of the heat transfer rod 104 is disposed in the introduction pipe 20. When the heat transfer rod 104 is cooled, the exhaled air that flows through the introduction pipe 20 is cooled, and the temperature of the exhaled air is reduced. The rod-shaped heat transfer rod 104 may be replaced by a plate-shaped, line-shaped, or spherical heat transfer member made of a thermally conductive material.

Since the exhaled air has a high humidity, vapor in the exhaled air cooled by the cooler 100 is easily saturated when the temperature is somewhat reduced, and starts to condense around fine particles in the exhaled air. As a result, the fine particles contained in the exhaled air are encapsulated in the droplets 3 and appear to be considerably greater in size. More specifically, when the temperature of the exhaled air is reduced, the exhaled air condenses around the target objects 1 in the exhaled air, so that the droplets 3 containing the target objects 1 are generated. The droplets 3 containing the target objects 1 flow together with the exhaled air that has not been formed into droplets, hit the rotating body 14 due to inertia, and are collected. Thus, the droplets 3 containing the target objects 1 are caused to flow into the space D in the main body 18 together with a part of the exhaled air introduced into the container 12 that has not been formed into droplets, and adhere to the rotating body 14 contained in the space D. More specifically, the droplets 3 containing the target objects 1 come into contact with the filter 46 filled with fibers, and are captured by the filter 46. The mechanism of collection is not limited to collection by inertia, and may instead be, for example, collection by diffusion.

The surface of each of the first blades 44a to 44h of the rotating body 14 may be either the filter 46 filled with fibers as described above or a simple plate surface. More specifically, each of the first blades 44a to 44h may have a plate surface that does not allow the droplets to pass therethrough in place of the filter 46. In this case, the droplets 3 come into contact with the plate surface and adhere to the plate surface, and the plate surface captures the droplets 3. Each of the first blades 44a to 44h may have both the filter 46 and the plate surface. More specifically, for example, the first blade 44a may have a filter in a radially outer region and a plate surface in a radially inner region. Each of the first blades 44b to 44h may have a filter in a radially outer region and a plate surface in a radially inner region. Thus, each of the first blades 44a to 44h includes at least one of the filter 46 and the plate surface, and the at least one of the filter 46 and the plate surface captures the droplets 3 generated by the cooler 100. For example, the plate surface may be a surface of a plate-shaped member made of a metal or a resin. Also in second to fifth embodiments described below, each filter 46 may be replaced by a plate surface as described above.

The rotating body 14 that has captured the droplets 3 rotates so that the droplets 3 are blown outward by centrifugal force, and the droplets 3 that have been blown outward adhere to the inner surface of the container 12 while the target objects 1 are contained therein. The droplets 3 that have adhered to the inner surface of the container 12 move toward a lower section of the container 12 due to gravity, and are mixed into the collection liquid 16. When the droplets 3 are mixed into the collection liquid 16, the target objects 1 contained in the droplets 3 are collected in the collection liquid 16. This also occurs when each filter 46 is replaced by a plate surface as described above. Thus, the target objects 1 contained in the exhaled air can be collected in the liquid.

When the rotating body 14 that has captured the droplets 3 rotates, the filters 46 at a position that is not immersed in the collection liquid 16 move to a position immersed in the collection liquid 16. When the filters 46 are immersed in the collection liquid 16, the droplets 3 that have not been blown and that remain captured on the filters 46 are mixed into the collection liquid 16, so that the target objects 1 contained in the droplets 3 are collected in the collection liquid 16. This also occurs when each filter 46 is replaced by a plate surface as described above. Thus, the target objects 1 contained in the exhaled air can be collected in the liquid.

In the first embodiment, the container 12 holds the collection liquid 16. However, the container 12 is not limited to this, and does not necessarily hold the collection liquid 16. In other words, the collection liquid 16 may not be contained in the container 12. In this case, the rotating body 14 that has been hit by the droplets 3 and captured the droplets 3 rotates so that the droplets 3 are removed therefrom, and the droplets 3 that have been removed accumulate in a bottom section (lower section) of the container 12 in the form of a solution containing fine particles (target objects 1). Thus, when the rotating body 14 that has captured the droplets 3 rotates, the droplets 3 captured by the rotating body 14 are blown outward by centrifugal force and accumulate in the lower section of the container 12. As the droplets 3 containing the target objects 1 accumulate in the lower section of the container 12, liquid containing the target objects 1 is obtained. In other words, the target objects 1 can be collected in the liquid. Thus, even when the container 12 does not hold the collection liquid 16, the target objects 1 in the exhaled air can be collected in liquid. Also in the second to fourth embodiments described below, it is not necessary that the container 12 (12a) hold the collection liquid 16.

A method for collecting the target objects 1 contained in the gas by using the collection device 10 according to the first embodiment will now be described. Here, a method for collecting the target objects 1 contained in air exhaled by a patient will be described.

First, the collection device 10 is attached to the patient. More specifically, the collection device 10 is attached to the patient so that the patient's mouth is covered with the conical tubular portion 40 of the introduction pipe 20. If the collection device 10 is attached to the patient with a gap left between the conical tubular portion 40 and the patient in the area around the patient's mouth, the air exhaled by the patient may leak to the outside. Therefore, there is a risk that a sufficient amount of pathogens cannot be collected from a small amount of air exhaled by the patient. Accordingly, a polyurethane sheet formed in the shape of a piece of tape may be attached to an opening 41 at the front of the conical tubular portion 40 to fill the gap between the conical tubular portion 40 and the patient in the area around the patient's mouth. To facilitate attachment of the collection device 10 to the patient, a connection portion between the conical tubular portion 40 and the main body 18, for example, the cylindrical portion 38, may be formed of a flexible tube made of rubber. In such a case, the orientation of the conical tubular portion 40 can be easily changed, so that the patient can reliably exhale air into the main body 18 of the collection device 10 without taking an uncomfortable position.

After the collection device 10 is attached to the patient, the patient inhales through the nose so that air is delivered to the lungs, and then exhales through the mouth. The exhaled air is introduced into the main body 18 through the introduction pipe 20. Although not described in detail herein, it is effective to check whether a required amount of exhaled air has been introduced into the container 12 by using, for example, a flowmeter and instruct the patient to exhale again if the required amount is not reached. The amount of exhaled air required for a test may be calculated in advance, and the result of the calculation may be used to reliably introduce the amount of exhaled air required for the test into the container 12.

As described above, the collection liquid 16 is contained in the main body 18 in advance. When, for example, the collection liquid 16 is a physiological saline solution, damage to the collected pathogens may be reduced. The amount of the collection liquid 16 is, for example, about 5 mL. The main body 18 may be fixed in a non-tiltable manner because the collection liquid 16 may leak from the main body 18 if the main body 18 is significantly tilted toward the introduction pipe 20 or the discharge pipe 22.

When the patient exhales, the rotating body 14 is rotated about the rotation axis X by the velocity pressure of the air exhaled by the patient, and the exhaled air passes through the filters 46 of the rotating body 14. A part of the air exhaled by the patient is cooled by the cooler 100 and formed into the droplets 3. The droplets 3 flow together with the exhaled air, come into contact with the filters 46, and are captured by the filters 46. In other words, the exhaled air comes into contact with the filters 46 while the droplets 3 are contained therein. The filters 46 may have a porosity less than or equal to a porosity at which the filters 46 receive a resistance by the velocity pressure of the exhaled air when the exhaled air is blown thereagainst. In such a case, the rotating body 14 can be easily rotated by the exhaled air.

As described below in the second embodiment, to ensure reliable rotation of the rotating body by the exhaled air, each of first blades 50a to 50h (described below) of a rotating body 14a (described below) may have a portion (contact portion 51 (described below)) that is in contact with an inner surface of a main body 18a defining the flow path A and that is composed of a flexible member made of silicone. The flexible member is disposed at a position adjusted so that the flexible member does not stop the rotation of the rotating body 14a and that a rotating force that rotates the rotating body 14a is not eliminated due to leakage of the exhaled air through a gap between the rotating body 14a and the main body 18a.

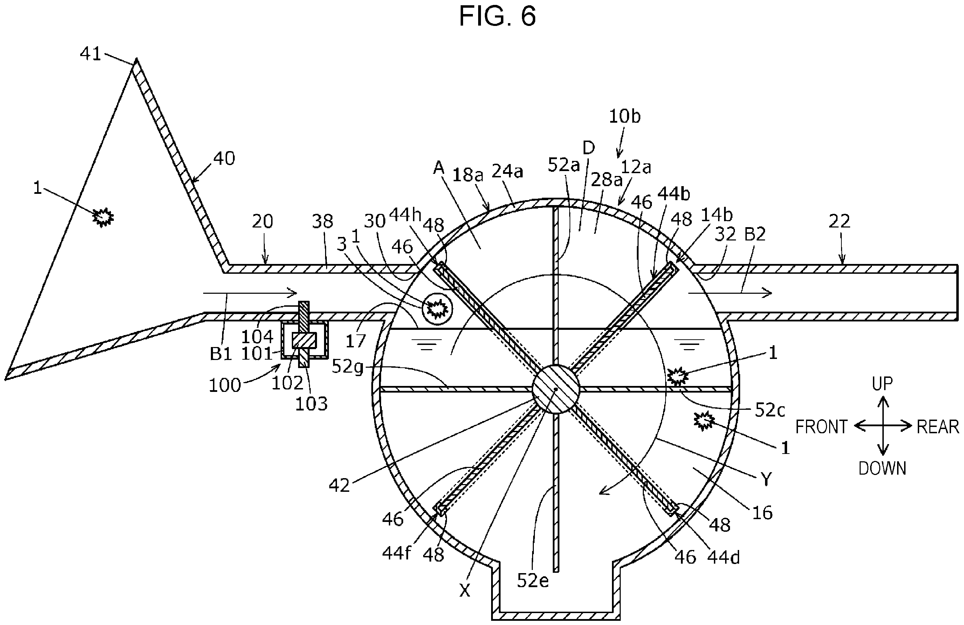

As described below in the third embodiment, to ensure sufficient rotating force for the rotating body, one or more first blades included in the first blades 44a to 44h may be replaced by one or more second blades (second blades 52a, 52c, 52e, and 52g in an example illustrated in Fig. 6). The material of each of the one or more second blades may be a plate member made of Teflon (registered trademark) that is less gas-permeable than the filters 46.

When the patient exhales air, the rotating body 14 rotates. The exhaled air flows toward the discharge pipe 22 while successively coming into contact with the filter 46 of the first blade 44a to the filter of the first blade 44h. When the rotating body 14 rotates, the first blades 44a to 44h successively move into the flow path A. Therefore, the exhaled air easily comes into contact with the first blades 44a to 44h and rotates the rotating body 14. The target objects 1, such as pathogens, contained in the exhaled air adhere to the surfaces of the filters 46. When the rotating body 14 rotates so that the filters 46 are immersed in the collection liquid 16, the target objects 1, such as pathogens, are isolated and collected in the collection liquid 16. The droplets 3 contained in the exhaled air adhere to the surfaces of the filters 46. When the rotating body 14 rotates, the droplets 3 are mixed into the collection liquid 16 after being removed by centrifugal force, or are mixed into the collection liquid 16 as a result of the filters 46 being immersed in the collection liquid 16. Thus, the target objects 1 contained in the droplets 3 are isolated and collected in the collection liquid 16.

Thus, when the introduced gas is the air exhaled by the patient, the droplets 3 are generated and the rotating body 14 disposed in the main body 18 is rotated in response to exhalation by the patient. If the air exhaled by the patient contains the target objects 1, such as pathogens, the target objects 1, such as pathogens, and/or the droplets 3 containing the target objects 1 are captured by the surfaces of the filters 46 of the rotating body 14 when the exhaled air passes through or comes into contact with the filters 46. As the rotating body 14 rotates, the captured target objects 1, such as pathogens, come into contact with the collection liquid 16 and leave the filters 46. Thus, the target objects 1 are isolated and collected in the collection liquid 16. Also, as the rotating body 14 rotates, the captured droplets 3 are mixed into the collection liquid 16 after being removed by centrifugal force, or are mixed into the collection liquid 16 as a result of the filters 46 being immersed in the collection liquid 16. Thus, the target objects 1 contained in the droplets 3 are isolated and collected in the collection liquid 16.

The target objects 1 contained in the gas can be collected in the liquid as described above.

Finally, the collection liquid 16 in which the target objects 1 are collected is recovered and tested for the presence or absence of a pathogen by using a pathogen detector. When the recovered collection liquid 16 contains pathogens, the pathogens can be extracted by the pathogen detector. The collection device 10 may be connected to the pathogen detector, and the operation from the introduction of the air exhaled by the patient to the test for determining the presence or absence of a pathogen may be automated.

As described above, the collection device 10 according to the present embodiment includes the container 12 into which the exhaled air containing the target objects 1 is introduced; the cooler 100 that reduces the temperature of the exhaled air introduced into the container 12 to generate the droplets 3 containing the target objects 1; and the rotating body 14 that is provided in the container 12 and that rotates. The rotating body 14 includes one or more first blades 44a to 44h which each project in a direction crossing the rotation axis X of the rotating body 14. Each of the one or more first blades 44a to 44h includes the filter 46 that captures the droplets 3.

Accordingly, the droplets 3 containing the target objects 1 in the exhaled air can be generated by the cooler 100. Since the rotating body 14 includes the filters 46 that capture the droplets 3, the generated droplets 3 can be captured by the rotating body 14. When the rotating body 14 rotates, the droplets 3 captured by the rotating body 14 can be blown outward by centrifugal force. The droplets 3 that have been blown accumulate in the container 12. Since the droplets 3 containing the target objects 1 accumulate in the container 12, liquid containing the target objects 1 can be obtained. Thus, the target objects 1 can be easily collected in the liquid by introducing the exhaled air.

The container 12 includes the main body 18 and the introduction pipe 20. The main body 18 has the space D therein that accommodates the rotating body 14. The introduction pipe 20 is connected to the main body 18 and communicates with the space D in the main body 18. The cooler 100 cools the exhaled air that flows through the introduction pipe 20.

Accordingly, the droplets 3 can be generated by cooling the exhaled air that flows through the introduction pipe 20, and the generated droplets 3 can be easily moved into the space D in the main body 18 together with the exhaled air. Since the rotating body 14 is disposed in the space D in the main body 18, the droplets 3 moved into the space D in the main body 18 can be easily captured by the rotating body 14.

The container 12 holds the collection liquid 16. When the rotating body 14 rotates, the filters 46 at a position that is not immersed in the collection liquid 16 move to a position immersed in the collection liquid 16.

Accordingly, the filters 46 can capture the droplets 3 contained in the exhaled air at a position that is not immersed in the collection liquid 16. When the filters 46 are immersed in the collection liquid 16, the droplets 3 captured by the filters 46 are mixed into the collection liquid 16, and the target objects 1 contained in the droplets 3 are collected in the collection liquid 16. Thus, the target objects 1 can be easily collected in the collection liquid 16.

### [Second Embodiment]

The second embodiment will now be described. The second embodiment mainly differs from the first embodiment in that the rotating body 14a rotates about the rotation axis X while an edge of each of the first blades 50a to 50h is in contact with the inner surface of a container 12a.

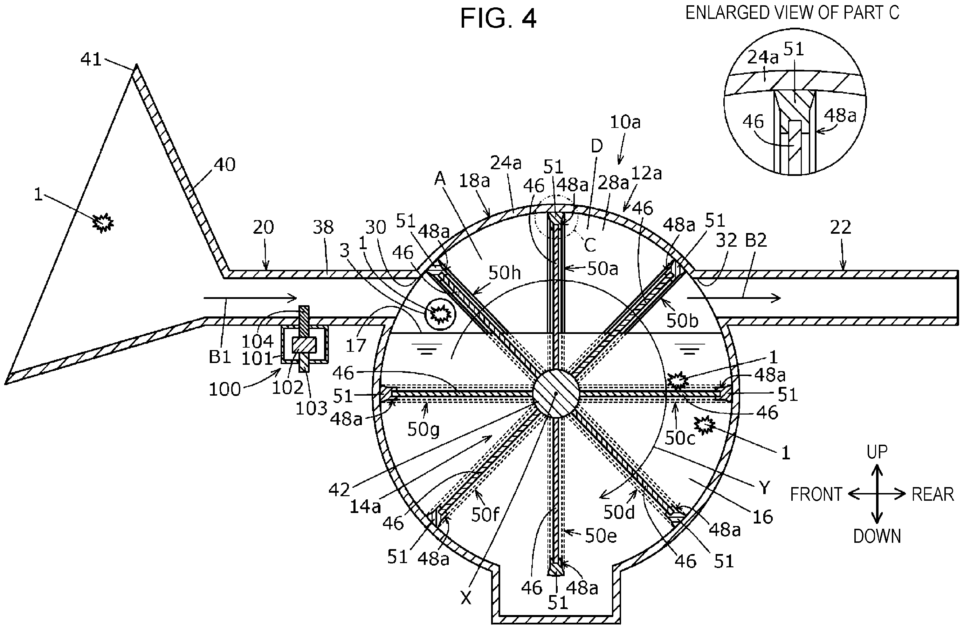

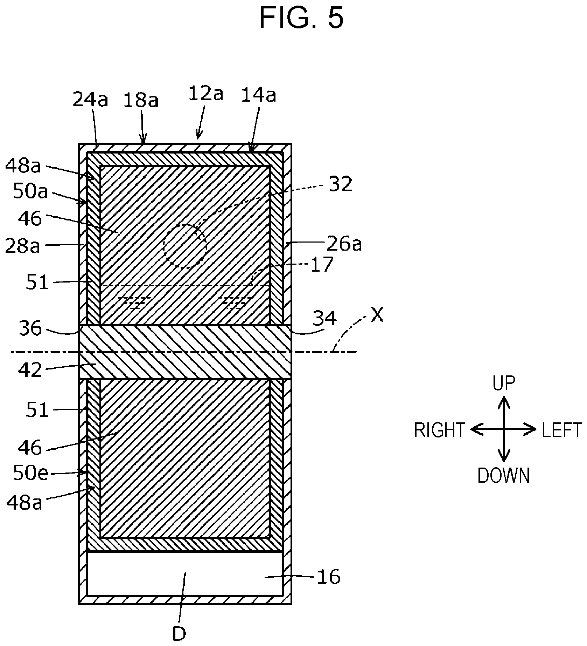

Fig. 4 is a sectional view of a collection device 10a according to the second embodiment as viewed from the left. Fig. 5 is a sectional view of the collection device 10a according to the second embodiment as viewed from the front. The collection device 10a according to the second embodiment will be described with reference to Figs. 4 and 5. The following description focuses on the differences from the collection device 10 according to the first embodiment.

The collection device 10a differs from the collection device 10 in that the collection device 10a includes the main body 18a that differs from the main body 18 and the first blades 50a to 50h that differ from the first blades 44a to 44h. The main body 18a differs from the main body 18 in that a lower section of the main body 18a is recessed downward. This facilitates collection of the target objects 1 contained in the collection liquid 16 in the lower section of the main body 18a. Each of the first blades 50a to 50h includes a frame 48a that differs from the frame 48 of each of the first blades 44a to 44h. The frame 48a includes the contact portion 51 that comes into contact with the inner surface of the main body 18a of the container 12a. The contact portion 51 is in contact with the inner surface of the main body 18a defining the flow path A when the contact portion 51 is positioned in the flow path A. The contact portion 51 is also in contact with the inner surface of the main body 18a when the contact portion 51 is immersed in the collection liquid 16. The inner surface of the main body 18a defining the flow path A is a portion of the inner surface of the main body 18a that is positioned above the liquid surface 17. The contact portion 51 is provided at an outer edge of the frame 48a. The contact portion 51 gradually widens outward and is in contact with the inner surface of the main body 18a. More specifically, in a direction perpendicular to the rotation axis X, the contact portion 51 gradually widens outward and is in contact with the inner surface of a peripheral wall 24a. In the left-right direction, the contact portion 51 gradually widens leftward and is in contact with the inner surface of a left wall 26a. In addition, in the left-right direction, the contact portion 51 gradually widens rightward and is in contact with the inner surface of a right wall 28a. The contact portion 51 may be, for example, a flexible member made of silicone. In the second embodiment, the rotating body 14a rotates about the rotation axis X while an edge (contact portion 51) of the first blade 50a is in contact with the inner surface of the main body 18a. The structure of each of the first blades 50b to 50h is similar to that of the first blade 50a. Therefore, the first blades 50b to 50h may be understood by referring to the above description of the first blade 50a, and detailed description thereof is thus omitted.

In the collection device 10a according to the present embodiment, the rotating body 14a rotates about the rotation axis X while an edge (contact portion 51) of each of the first blades 50a to 50h is in contact with the inner surface of the container 12a.

Accordingly, the rotating body 14a rotates about the rotation axis X while an edge (contact portion 51) of each of the first blades 50a to 50h is in contact with the inner surface of the main body 18a of the container 12a. When each of the first blades 50a to 50h has the contact portion 51 at an edge thereof as described above, the gas that flows through the container 12a (flow path A) can be prevented from flowing through the gaps between the main body 18a and the rotating body 14a. Accordingly, the gas that flows through the container 12a (flow path A) easily passes through the filters 46, and the target objects 1 and the droplets 3 containing the target objects 1 in the gas can be more reliably captured. In other words, the gas that flows through the container 12a (flow path A) can be prevented from being discharged without passing through the filters 46. In the second embodiment illustrated in Figs. 4 and 5, the frame 48a is provided with the contact portion 51 at the edges thereof in the direction perpendicular to the rotation axis X and in the left-right direction. However, the frame 48a may instead have the contact portion 51 at the edge thereof in the direction perpendicular to the rotation axis X or at the edges thereof in the left-right direction as long as the contact portion 51 does not inhibit the rotation of the rotating body 14a. It is not necessary that the contact portion 51 gradually widen outward.

### [Third Embodiment]

The third embodiment will now be described. The third embodiment mainly differs from the first embodiment in that the second blades 52a, 52c, 52e, and 52g are provided in place of the first blades 44a, 44c, 44e, and 44g.

Fig. 6 is a sectional view of a collection device 10b according to the third embodiment as viewed from the left. The collection device 10b according to the third embodiment will be described with reference to Fig. 6. The following description focuses on differences from the collection device 10 according to the first embodiment.

The collection device 10b differs from the collection device 10 according to the first embodiment in that the collection device 10b includes the container 12a in place of the container 12. The collection device 10b also differs from the collection device 10 according to the first embodiment in that the collection device 10b includes a rotating body 14b in place of the rotating body 14. The container 12a may be understood by referring to the above description of the second embodiment, and detailed description thereof is thus omitted. The rotating body 14b differs from the rotating body 14 in that the rotating body 14b includes the second blade 52a in place of the first blade 44a, the second blade 52c in place of the first blade 44c, the second blade 52e in place of the first blade 44e, and the second blade 52g in place of the first blade 44g. The second blades 52a, 52c, 52e, and 52g project in directions crossing the rotation axis X, and are arranged at equal intervals around the rotation axis X. In other words, the second blades 52a, 52c, 52e, and 52g project radially outward with respect to the shaft 42 from the outer peripheral surface of the shaft 42, and are attached to the outer peripheral surface of the shaft 42. The second blade 52a extends in the left-right direction and in a direction perpendicular to the rotation axis X, and is substantially rectangular-plate-shaped. The second blade 52a is in contact with the inner surface of the main body 18a of the container 12a. More specifically, the second blade 52a is in contact with the inner surface of the main body 18a defining the flow path A when the second blade 52a is positioned in the flow path A. The second blade 52a is also in contact with the inner surface of the main body 18a when the second blade 52a is immersed in the collection liquid 16. More specifically, the second blade 52a is in contact with the inner surface of the peripheral wall 24a at an edge thereof in a direction perpendicular to the rotation axis X. The second blade 52a is in contact with the inner surface of the left wall 26a (see Fig. 5) at an edge thereof in the left-right direction. The second blade 52a is in contact with the inner surface of the right wall 28a at an edge thereof in the left-right direction. The second blade 52a is arranged together with the first blades 44b, 44d, 44f, and 44h around the rotation axis X. When the rotating body 14b rotates about the rotation axis X, the second blade 52a also rotates about the rotation axis X. The rotating body 14b rotates about the rotation axis X while the edges of the second blade 52a are in contact with the inner surface of the main body 18a. The material of the second blade 52a may be a resin, such as Teflon (registered trademark), and the second blade 52a is less gas-permeable than the filters 46. The second blade 52a may not be gas permeable. The structures of the second blades 52c, 52e, and 52g are similar to that of the second blade 52a. Therefore, the second blades 52c, 52e, and 52g may be understood by referring to the above description of the second blade 52a, and detailed description thereof is thus omitted.

Each of the first blades 44b, 44d, 44f, and 44h may be in contact with the inner surface of the main body 18a of the container 12a. In such a case, the rotating body 14b rotates about the rotation axis X while each of the first blades 44b, 44d, 44f, and 44h is in contact with the inner surface of the main body 18a of the container 12a.

As described above, in the collection device 10b according to the present embodiment, the rotating body 14b includes the second blades 52a, 52c, 52e, and 52g that project in directions crossing the rotation axis X and that are less gas-permeable than the filters 46.

Thus, the rotating body 14b includes the second blades 52a, 52c, 52e, and 52g that are less gas-permeable than the filters 46. When, for example, the gas that is introduced is air exhaled by the patient, since the second blades 52a, 52c, 52e, and 52g that are less gas-permeable than the filters 46 are provided, the rotating body 14b easily receives force from the exhaled air that is introduced. Accordingly, the rotating body 14b can be easily rotated. Therefore, the target objects 1 and the droplets 3 containing the target objects 1 in the gas can be easily captured, and the target objects 1 can be more easily collected in the collection liquid 16. The number of second blades may be any number as long as the rotating body 14b can be rotated by the exhaled air and the pathogens in the exhaled air can be captured by the filters 46.

### [Fourth Embodiment]

The fourth embodiment will now be described. The fourth embodiment mainly differs from the first embodiment in that a driving device 54 is additionally provided.

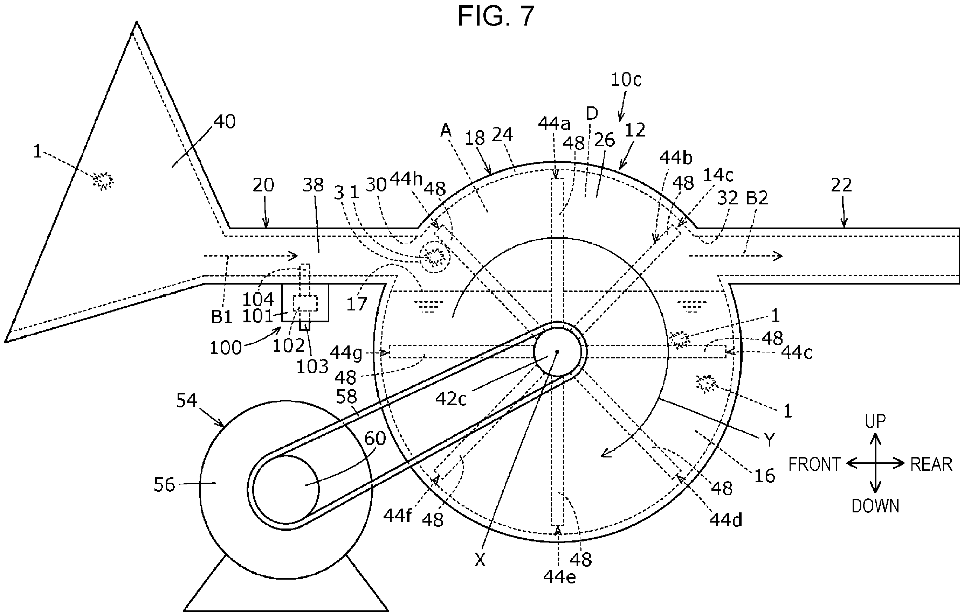

Fig. 7 is a side view of a collection device 10c according to the fourth embodiment as viewed from the left. The collection device 10c according to the fourth embodiment will be described with reference to Fig. 7. The following description focuses on differences from the collection device 10 according to the first embodiment.

The collection device 10c differs from the collection device 10 in that the collection device 10c additionally includes the driving device 54. The collection device 10c also differs from the collection device 10 in that a shaft 42c projects outward from the left wall 26 in the left-right direction. The driving device 54 applies rotational force to a rotating body 14c and rotates the rotating body 14c about the rotation axis X. The driving device 54 includes a motor 56 and a belt 58. The belt 58 is wrapped around the shaft 42c and a rotation shaft 60 of the motor 56. When the motor 56 is driven, the shaft 42c is rotated by the belt 58 so that the rotating body 14c rotates. The rotating body 14c includes the first blades 44a to 44h. However, a rotating body may include a single first blade.

As described above, the collection device 10c according to the present embodiment additionally includes the driving device 54 that rotates the rotating body 14c about the rotation axis X.

Accordingly, when, for example, the gas that is introduced is indoor air, the rotating body 14c can be easily rotated by connecting the motor 56 to the shaft 42c of the rotating body 14c and transmitting the rotation of the motor 56 to the shaft 42c of the rotating body 14c. Therefore, ambient air can be easily introduced into the flow path A, and the target objects 1, such as pathogens, contained in the ambient air can be easily isolated and collected in the collection liquid 16. When the rotating body 14c is continuously rotated by the driving device 54, the gas can be efficiently introduced, and the target objects 1 in the gas can be efficiently collected. When exhaled air is introduced, it is not necessary to blow the exhaled air against the rotating body 14c to rotate the rotating body 14c, and the rotating body 14c can be easily rotated by the driving device 54. Therefore, the droplets 3 can be easily captured, and the target objects 1 contained in the droplets 3 can be easily collected in the liquid.

In any of the embodiments described above with reference to Figs. 1 to 7, the gas (exhaled air) is introduced to capture the target objects, such as pathogens, and the droplets containing the target objects with the filters of the rotating body. The rotating body is rotated so that the target objects, such as pathogens, are isolated and collected in the collection liquid. After that, the collection liquid may be extracted and subjected to a test for the pathogens. Although not illustrated in the drawings, a pipe for extracting the collection liquid may be provided on a lower section of the main body. A pump, for example, may be used to deliver the collection liquid to a pathogen testing device provided in combination with the collection device, and the number and/or type of the pathogens may be determined. Accordingly, for example, measures can be quickly taken to prevent secondary infections by the pathogens.

### [Fifth Embodiment]

The fifth embodiment will now be described.

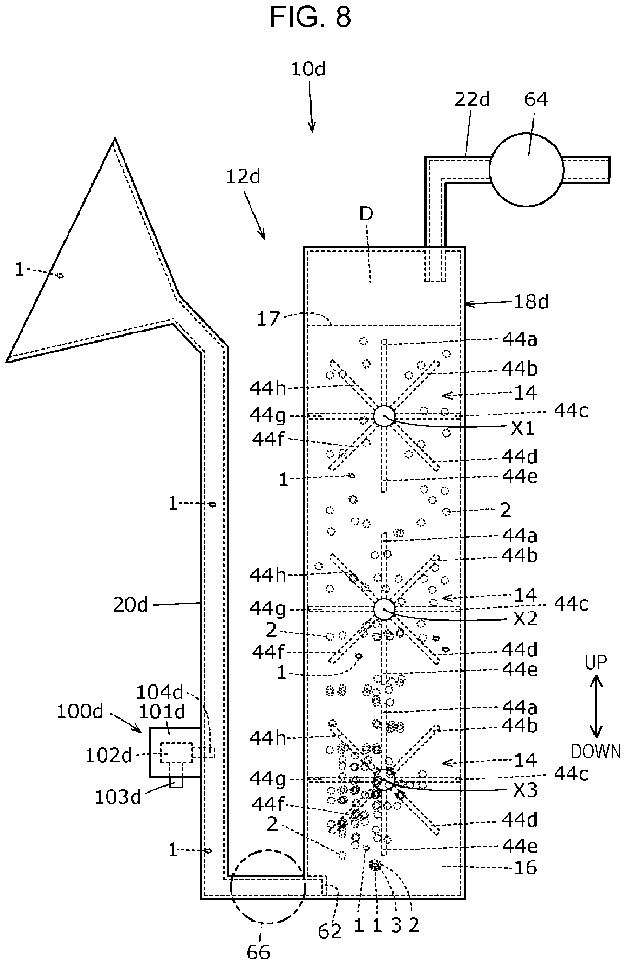

Fig. 8 is a side view of a collection device 10d according to the fifth embodiment. The collection device 10d according to the fifth embodiment will be described with reference to Fig. 8. In this embodiment, a direction from a main body 18d (described below) toward a location at which an introduction pipe 20d (described below) is connected is referred to as downward, and a direction from the main body 18d toward a location at which a discharge pipe 22d (described below) is connected is referred to as upward (see arrows in Fig. 8). In Fig. 8, the downward direction may be a vertical direction.

Referring to Fig. 8, the collection device 10d collects target objects 1 contained in exhaled air in liquid. The collection device 10d includes a container 12d, three rotating bodies 14, and collection liquid 16. Each component will be described.

The container 12d holds the collection liquid 16 therein. The container 12d supports the three rotating bodies 14 such that the three rotating bodies 14 are rotatable about rotation axes X1, X2, and X3 that extend perpendicularly to the up-down direction. The rotation axes X1, X2, and X3 extend in the same direction and are spaced from each other in the up-down direction. The rotation axes X1, X2, and X3 do not necessarily extend perpendicularly to the up-down direction as long as the rotation axes X1, X2, and X3 extend in directions crossing the up-down direction. The container 12d includes the main body 18d, the introduction pipe 20d, and the discharge pipe 22d.

The main body 18d holds the collection liquid 16 and supports the three rotating bodies 14 such that the three rotating bodies 14 are rotatable about the rotation axes X1, X2, and X3. The main body 18d is substantially rectangular-parallelepiped-shaped, and has a space D therein that accommodates the collection liquid 16 and the three rotating bodies 14. The main body 18d has an inlet 62 through which gas flows into the collection liquid 16 at a location below the three rotating bodies 14. The inlet 62 is provided in a lower section of the main body 18d. The inlet 62 is provided with, for example, a check valve that allows the gas to flow into the collection liquid 16 from the introduction pipe 20d but does not allow the collection liquid 16 to flow into the introduction pipe 20d.

Air exhaled by a user of the collection device 10d or air surrounding the collection device 10d is introduced through the introduction pipe 20d into the collection liquid 16 at a location below the three rotating bodies 14. The introduction pipe 20d is connected to the inlet 62 of the main body 18d. The gas, such as the exhaled air, introduced through the introduction pipe 20d is discharged through the discharge pipe 22d. The discharge pipe 22d is connected to an upper section of the main body 18d. The discharge pipe 22d communicates with a portion of the space D in the main body 18d in a region above the collection liquid 16.

The three rotating bodies 14 are immersed in the collection liquid 16 in the container 12d, and are arranged next to each other in the up-down direction. The rotating body 14 at the top is supported by the main body 18d such that the rotating body 14 at the top is rotatable about the rotation axis X1. The rotating body 14 in the middle is supported by the main body 18d such that the rotating body 14 in the middle is rotatable about the rotation axis X2. The rotating body 14 at the bottom is supported by the main body 18d such that the rotating body 14 at the bottom is rotatable about the rotation axis X3. The three rotating bodies 14 may be understood by referring to the above description of the first embodiment, and detailed description thereof is thus omitted. Each of the three rotating bodies 14 includes the first blades 44a to 44h, and the first blades 44a to 44h include the respective filters 46 (see Figs. 1 to 3). The filters 46 capture the target objects 1 in the gas 2 by coming into contact with gas 2 that has flowed into the collection liquid 16 from the introduction pipe 20d (inlet 62) and that moves upward through the collection liquid 16. As described in detail below, the droplets 3 generated by a cooler 100d are contained in the exhaled air that flows through the introduction pipe 20d, and are introduced into the collection liquid 16. The filters 46 (see Figs. 1 to 3) capture the droplets 3 in the gas 2 by coming into contact with the gas 2, which is the exhaled air that has been introduced into the collection liquid 16 from the introduction pipe 20d (inlet 62) and that moves upward through the collection liquid 16.

The rotating body 14 at the bottom rotates about the rotation axis X3 when the gas 2 that moves through the collection liquid 16 comes into contact with any one of the first blades 44a to 44h of the rotating body 14 at the bottom. When the rotating body 14 at the bottom rotates about the rotation axis X3, the target objects 1 captured by the filters 46 included in the rotating body 14 at the bottom are collected in the collection liquid 16. In addition, when the rotating body 14 at the bottom rotates about the rotation axis X3, the droplets 3 captured by the filters 46 included in the rotating body 14 at the bottom are mixed into the collection liquid 16, so that the target objects 1 contained in the droplets 3 are collected in the collection liquid 16. The gas 2 that has flowed into the collection liquid 16 is in the form of, for example, bubbles.

The rotating body 14 in the middle rotates about the rotation axis X2 when the gas 2 that moves through the collection liquid 16 comes into contact with any one of the first blades 44a to 44h of the rotating body 14 in the middle. When the rotating body 14 in the middle rotates about the rotation axis X2, the target objects 1 captured by the filters 46 included in the rotating body 14 in the middle are collected in the collection liquid 16. In addition, when the rotating body 14 in the middle rotates about the rotation axis X2, the droplets 3 captured by the filters 46 included in the rotating body 14 in the middle are mixed into the collection liquid 16, so that the target objects 1 contained in the droplets 3 are collected in the collection liquid 16. The gas 2 that has flowed into the collection liquid 16 is in the form of, for example, bubbles.

The rotating body 14 at the top rotates about the rotation axis X1 when the gas 2 that moves through the collection liquid 16 comes into contact with any one of the first blades 44a to 44h of the rotating body 14 at the top. When the rotating body 14 at the top rotates about the rotation axis X1, the target objects 1 captured by the filters 46 included in the rotating body 14 at the top are collected in the collection liquid 16. In addition, when the rotating body 14 at the top rotates about the rotation axis X1, the droplets 3 captured by the filters 46 included in the rotating body 14 at the top are mixed into the collection liquid 16, so that the target objects 1 contained in the droplets 3 are collected in the collection liquid 16. The gas 2 that has flowed into the collection liquid 16 is in the form of, for example, bubbles.

The collection liquid 16 is used to collect the target objects 1, and is contained in the main body 18d. More specifically, the collection liquid 16 is contained in the main body 18d such that the liquid surface 17 of the collection liquid 16 is positioned above the rotating body 14 at the top.

The cooler 100d reduces the temperature of the exhaled air that flows through the introduction pipe 20d so that the exhaled air is formed into droplets. Thus, the cooler 100d generates the droplets 3 containing the target objects 1. The cooler 100d includes a housing 101 d, a cooling source 102d, a power supply switch 103d, and a heat transfer rod 104d.

The housing 101d is attached to the introduction pipe 20d. The cooling source 102d is contained in the housing 101d. The cooling source 102d may be understood by referring to the above description of the cooling source 102. The power supply switch 103d may be understood by referring to the above description of the power supply switch 103. The heat transfer rod 104d may be understood by referring to the above description of the heat transfer rod 104. Therefore, detailed description of these components will be omitted.

When the patient exhales air, a part of the exhaled air is cooled by the cooler 100d and formed into the droplets 3. The droplets 3 are introduced into the collection liquid 16 together with a part of the exhaled air that has flowed through the introduction pipe 20d without being formed into droplets. As described above, the droplets 3 that have been introduced are captured by the filters 46 of the rotating bodies 14 and mixed with the collection liquid 16 so that the target objects 1 contained in the droplets 3 are collected in the collection liquid 16.

Although the main body 18d of the collection device 10d contains three rotating bodies 14, this is because a small amount of pathogens can be efficiently collected by installing more than one rotating bodies 14. Therefore, it is not necessary that the number of rotating bodies 14 be three.

A phosphate buffer solution having a concentration adjusted to a predetermined concentration may be poured into the main body 18d as the collection liquid 16. The amount of phosphate buffer solution poured into the main body 18d may be such that all of the rotating bodies 14 disposed in the main body 18d are immersed in the phosphate buffer solution. A connection location at which the introduction pipe 20d is connected to the main body 18d may be on a side surface of a lowermost section or a bottom section of the main body 18d. The connection location may be positioned such that the gas introduced through the introduction pipe 20d efficiently comes into contact with any of the first blades 44a to 44h included in each of the rotating bodies 14 installed in the main body 18d.

When the amount of pathogens contained in the gas is extremely small, more than one rotating bodies 14 may be installed as illustrated in Fig. 8. In this case, when the inlet 62 is provided in the lowermost section of the main body 18d that holds the collection liquid 16, the gas containing the pathogens comes into contact with the filters 46 of the rotating bodies 14 more than once. Accordingly, the pathogens are captured by the filters 46, and are isolated and collected in the collection liquid 16 as the rotating bodies 14 rotate. When the gas is discharged by using a pump 64 provided on the discharge pipe 22d in the upper section of the main body 18d, gas can be efficiently introduced into the main body 18d through the introduction pipe 20d.

A method for collecting the target objects 1 contained in the gas by using the collection device 10d according to the fifth embodiment will now be described.

When the target objects 1 are collected from the air exhaled by the patient, the patient exhales air into the introduction pipe 20d, and the filters 46 capture the target objects 1, such as pathogens, and the droplets 3 containing the target objects 1. As the rotating bodies 14 rotate, the target objects 1, such as pathogens, are isolated and collected in the phosphate buffer solution.

When the target objects 1, such as pathogens, contained in the atmosphere are to be collected instead of the target objects 1 in the exhaled air, a pump 66, for example, may be provided on the introduction pipe 20d of the collection device 10d. The pump 66 may be activated to introduce the gas into the collection liquid 16. The type and model of the pump 66 are not limited as long as the target gas can be introduced into the collection liquid 16. The phosphate buffer solution (collection liquid 16) in which the target objects 1, such as pathogens, are collected is recovered and analyzed by using a pathogen detector. When the recovered phosphate buffer solution contains pathogens, the pathogens can be extracted by the pathogen detector.

As described above, the collection device 10d according to the present embodiment includes the main body 18d that holds the collection liquid 16; the rotating body 14 that is immersed in the collection liquid 16 in the main body 18d and that rotates about the rotation axis X1 extending in a direction crossing the up-down direction; the introduction pipe 20d through which the droplets 3 containing the target objects 1 and first gas are introduced into the collection liquid 16 at a location below the rotating body 14; and the cooler 100d that reduces the temperature of gas introduced into the introduction pipe 20d and that generates the droplets 3 and the first gas. The rotating body 14 includes one or more first blades 44a to 44h which each project in a direction crossing the rotation axis X1 of the rotating body 14. Each of the one or more first blades 44a to 44h includes the filter 46. After the droplets 3 and the first gas are introduced into the collection liquid 16 through the introduction pipe 20d, the filter 46 comes into contact with the first gas that moves upward through the collection liquid 16 along with the droplets 3, and captures the droplets 3.

Accordingly, the droplets 3 containing the target objects 1 and the first gas are introduced into the collection liquid 16 at the location below the rotating body 14, and the first gas moves upward along with the droplets 3. Then, the filters 46 of the rotating body 14 come into contact with the first gas that moves upward along with the droplets 3, and thereby capture the droplets 3. The rotating body 14 rotates about the rotation axis X3 when the first gas that moves through the collection liquid 16 along with the droplets 3 comes into contact with any one of the first blades 44a to 44h. When the rotating body 14 rotates about the rotation axis X3, the droplets 3 captured by the filters 46 are mixed into the collection liquid 16, so that the target objects 1 contained in the droplets 3 are collected in the collection liquid 16. Thus, when the gas is introduced into the introduction pipe 20d, the target objects 1 contained in the gas can be easily collected in the collection liquid 16. The first gas that moves through the collection liquid 16 along with the droplets 3 forms small bubbles when the first gas comes into contact with the rotating body 14 that rotates about the rotation axis X3. Therefore, the droplets 3 can be easily captured by the filters 46. Thus, the target objects 1 in the gas can be more reliably collected.

Although collection devices according to one or more aspects of the present disclosure have been described with reference to the embodiments, the present disclosure is not limited to these embodiments. The scope of one or more aspects of the present disclosure may include embodiments obtained by applying various modifications conceivable by those skilled in the art to the above-described embodiments and embodiments obtained by combining constituent elements in different ones of the above-described embodiments as long as they do not depart from the spirit of the present disclosure.

In the above-described embodiments, the cooler 100 cools the exhaled air that flows through the introduction pipe 20 to generate the droplets 3, and the cooler 100d cools the exhaled air that flows through the introduction pipe 20d to generate the droplets 3. However, the coolers are not limited to this. For example, a cooler may cool the exhaled air in a main body of a container to generate the droplets 3. More specifically, for example, a heat transfer rod may be provided in the main body of the container such that the heat transfer rod does not impede rotation of a rotating body, and the cooler may cool the heat transfer rod to cool the exhaled air in the main body of the container and generate the droplets 3.

In the above-described embodiments, the cooler 100 cools the heat transfer rod 104 positioned in the introduction pipe 20 to cool the exhaled air that flows through the introduction pipe 20, and the cooler 100d cools the heat transfer rod 104d positioned in the introduction pipe 20d to cool the exhaled air that flows through the introduction pipe 20d. However, the coolers are not limited to this. For example, a cooler may cool the exhaled air in a container by cooling the container. When, for example, the container is thermally conductive, the temperature of the inner surface of the container can be reduced by cooling the outer surface of the container, and accordingly the temperature of the exhaled air in the container can be reduced. For example, the container can be cooled by bringing a cooling source, such as ice, into contact with the outer surface of the container.

In the above-described embodiments, the cylindrical portion 38 and the discharge pipe 22 extend in the front-rear direction. However, for example, the cylindrical portion 38 may extend diagonally upward and forward, and the discharge pipe 22 may extend diagonally upward and rearward.

In the above-described embodiments, the liquid surface 17 of the collection liquid 16 is formed above the rotation axis X. However, the liquid surface 17 is not limited to this. For example, the liquid surface 17 of the collection liquid 16 may be formed below the rotation axis X.

A collection device according to an aspect of the present disclosure may include a vibrator for separating the target objects 1 captured by the filters 46 from the filters 46 and facilitating collection of the target objects 1 in the collection liquid 16. The vibrator is, for example, an ultrasonic vibrator, and is electrically connected to a power supply. When a power supply switch is turned on, the vibrator vibrates to generate ultrasonic waves. The vibrator is provided in the container 12 and immersed in the collection liquid 16 contained in the container 12. When the vibrator vibrates, vibration of the vibrator is transmitted to the collection liquid 16, and the collection liquid 16 vibrates. Examples of the vibrator include a crystal vibrator, a SAW resonator, a MEMS vibrator, and other piezoelectric vibrators.

### Industrial Applicability

The present disclosure is widely applicable to devices that collect aerosols from air.

### Reference Signs List

10, 10a, 10b, 10c, 10d collection device
12, 12a, 12d container
14, 14a, 14b, 14c rotating body
16 collection liquid
17 liquid surface
18, 18a, 18d main body
20, 20d introduction pipe
22, 22d discharge pipe
24, 24a peripheral wall
26, 26a left wall
28, 28a right wall
30, 32, 34, 36 hole
38 cylindrical portion
40 conical tubular portion
42, 42c shaft
44a, 44b, 44c, 44d, 44e, 44f, 44g, 44h, 50a, 50b, 50c, 50d, 50e, 50f, 50g, 50h first blade
46 filter
48, 48a frame
51 contact portion
52a, 52c, 52e, 52g second blade
54 driving device
56 motor
58 belt
60 rotation shaft
62 inlet
64, 66 pump
100, 100d cooler
101, 101d housing
102, 102d cooling source
103, 103d power supply switch
104, 104d heat transfer rod

## Claims

1. A collection device comprising:
a container into which exhaled air containing target objects is introduced;
a cooler that reduces a temperature of the exhaled air introduced into the container to generate droplets containing the target objects; and
a rotating body that is provided in the container and that rotates,
wherein the rotating body includes a first blade that projects in a direction crossing a rotation axis of the rotating body, and
wherein the first blade includes at least one of a filter that captures the droplets and a plate surface that does not allow the droplets to pass therethrough.

2. The collection device according to Claim 1, wherein the container includes a main body and an introduction pipe, the main body having a space therein that accommodates the rotating body, the introduction pipe being connected to the main body and communicating with the space in the main body, and
wherein the cooler cools the exhaled air that flows through the introduction pipe.

3. The collection device according to Claim 1 or 2, wherein the container holds the collection liquid, and
wherein when the rotating body rotates, the at least one of the filter and the plate surface moves from a position that is not immersed in the collection liquid to a position immersed in the collection liquid.

4. The collection device according to any one of Claims 1 to 3, wherein the rotating body rotates about the rotation axis while an edge of the first blade is in contact with an inner surface of the container.

5. The collection device according to any one of Claims 1 to 4, wherein the first blade includes the filter, and
wherein the rotating body further includes a second blade that projects in a direction crossing the rotation axis, the second blade being less permeable to the exhaled air than the filter.

6. The collection device according to any one of Claims 1 to 5, further comprising a driving device that rotates the rotating body about the rotation axis.

7. A collection device comprising:
a main body that holds collection liquid;
a rotating body that is immersed in the collection liquid in the main body and that rotates about a rotation axis extending in a direction crossing an up-down direction;
an introduction pipe through which droplets containing target objects and first gas are introduced into the collection liquid from a location below the rotating body; and
a cooler that reduces a temperature of gas introduced into the introduction pipe and that generates the droplets and the first gas,
wherein the rotating body includes a first blade that projects in a direction crossing the rotation axis,
wherein the first blade includes a filter, and
wherein after the droplets and the first gas are introduced into the collection liquid through the introduction pipe, the filter comes into contact with that moves upward through the collection liquid along with the droplets and captures the droplets.
